# EUROPÄISCHE PATENTSCHRIFT

(11) **EP 1 883 474 B1**
(45) Veröffentlichungstag und Bekanntmachung des Hinweises auf die Patenterteilung: **31.03.2021**
(21) Anmeldenummer: 06763221.6
(22) Anmeldetag: 22.05.2006
(51) Int. Cl.: B01L 3/00, B01L 7/00, C12Q 1/686, G01N 35/08, G01N 35/10, B01L 9/00, G01N 35/00

(54) **SYSTEM ZUR INTEGRIERTEN UND AUTOMATISIERTEN DNA- ODER PROTEIN-ANALYSE UND BETRIEBSVERFAHREN EINES SOLCHEN SYSTEMS**
SYSTEM FOR THE INTEGRATED AND AUTOMATED ANALYSIS OF DNA OR PROTEIN AND METHOD FOR OPERATING SAID TYPE OF SYSTEM
SYSTEME D'ANALYSE D'ADN OU DE PROTEINES INTEGREE ET AUTOMATISEE ET PROCEDE DE FONCTIONNEMENT D'UN TEL SYSTEME

(30) Priorität: 25.05.2005 DE 102005024610
(43) Veröffentlichungstag der Anmeldung: 06.02.2008
(73) Patentinhaber: Boehringer Ingelheim Vetmedica GmbH, 55216 Ingelheim am Rhein (DE)
(72) Erfinder: PAULICKA, Peter, 91056 Erlangen (DE); GUMBRECHT, Walter, 91074 Herzogenaurach (DE)
(74) Vertreter: Von Rohr Patentanwälte Partnerschaft mbB
(86) Internationale Anmeldenummer: PCT/EP2006/062499
(87) Internationale Veröffentlichungsnummer: WO 2006/125767

(56) Entgegenhaltungen:
- EP-A- 1 418 243
- EP-A- 1 473 085
- WO-A-02/072262
- US-A- 5 755 942
- US-A1- 2002 142 482

## Beschreibung

Die Erfindung bezieht sich auf ein System zur integrierten und automatisierten DNA- oder Protein-Analyse mit einer einmal verwendbaren Cartridge (Karte), einem Auswertegerät sowie Mitteln zur Signalaufnahme und -verarbeitung, wobei das Auswertegerät ein Steuergerät zur Ausführung eines Analyseprozesses bei in das Auswertegerät eingebrachter Cartridge enthält. Daneben bezieht sich die Erfindung auch auf Verfahren zum Betrieb des Steuergerätes

Die Bedeutung von dezentralen Analysegeräten ("Point of Care", "Near Patient Testing") in der Molekulardiagnostik bzw. allgemeine Medizintechnik wächst. Daneben werden Schnelltests in den Bereichen Lebensmittelüberwachung und Umweltschutz immer wichtiger.

In der allgemeinen, aber auch speziell in der Patentliteratur liegt eine Vielzahl von Veröffentlichungen zu vorgenanntem Thema vor: Aus der WO 02/072 262 A1 und der WO 02/073 153 A1 sind Systeme zur Anwendung in der biomedizinischen Technik bekannt, bei dem dezentral genommene Proben dezentral mit einem ggf. mobilen Auslesegerät ausgewertet werden. Insbesondere die Proben werden dabei in einer einmal verwendbaren scheckkartenförmigen Cartridge ("Karte") gesammelt. Eine solche Cartridge zur Anwendung bei der DNA-Analyse aus Vollblutproben ist im Einzelnen in der nicht vorveröffentlichten WO 2006/042 838 A1 offenbart. Dabei werden folgende Merkmale der Cartridge als wesentlich zur automatischen Durchführung aller für eine Prozessanalytik aus einzelnen Teilprozessen in der Cartridge notwendigen Maßnahmen angesehen:
- In der Cartridge ist ein System von Mikrokanälen und/oder Mikrokavitäten für eine mikrofluidische Prozesstechnik vorhanden,
- die Mikrokanäle bzw. die Mikrokavitäten weisen vorgegebene geometrische Strukturen auf, um Reagenzien aufzunehmen, wobei
- die Reagenzien an bestimmten Stellen in den Mikrokanälen bzw. die Mikrokavitäten der Cartridge in einer lagerstabilen Form bevorratet sind,
- es sind Mittel vorhanden, um die trockengelagerten Reagenzien für den jeweiligen Teilprozess in geeigneter Form, insbesondere als flüssiges Reagenz, bereit zu stellen.

Weiterhin sind in der US 2002/0 179 444 A1 und in der US 2003/0 148 530 A1 Einzellösungen für ein Blut-Diagnose-Verfahren und die zugehörige Signalverarbeitung bekannt.

Die EP 1 473 085 A1 offenbart eine Cartridge für biochemische Reaktionen, die eine Probe aufnehmen und zur Durchführung der Reaktion in ein Analysegerät eingesetzt werden kann.

Die US 5,755,942 A offenbart ein System zur parallelen Durchführung einer Vielzahl von Tests. Das System hat ein Mikro-Array, einen Probenkanal zum Zuführen der Proben zu dem Array und eine Steuervorrichtung, die die Flüssigkeitszufuhr zu dem Array überwachen und steuern kann.

Die US 2002/142482 A1 offenbart ein mikrofluidisches System zur Untersuchung von Flüssigkeiten, die biologische Zellen enthalten. Das System umfasst eine Cartridge zur Aufnahme einer Probe, wobei die Analyse von einem Computer gesteuert wird.

Ausgehend von obigem Stand der Technik liegt der Erfindung die Aufgabe zugrunde, ein System der eingangs genannten Art zu schaffen, das insbesondere bezüglich des Auswertegerätes den Anforderungen der Praxis genügt, und weiterhin ein zugehöriges Steuerverfahren für die Auswertung bei einem System aus Cartridge und Auswertegerät anzugeben. Dabei werden folgende Anforderungen an das Auswertegerät gestellt:
- Niedriger Preis
- kleine Bauform
- einfache Bedienung ("one button"-Betrieb)
- schnelle Durchführung der Analyse
- integrierte Mikrofluidik
- Kontaminationssicherheit, d.h. Vermeidung von Verschmutzungen und Infektionen
- hohe Betriebssicherheit
- Kommunikationsfähigkeit mit Datenverarbeitungs-Systemen.

Es ist bisher keine praxisgerechte Lösung zur vollautomatischen Nukleinsäure-Analyse der Probe (z.B. Blut) bis zur Ergebnis-Darstellung, insbesondere inklusive Zellaufschluss, PCR und Detektion, bekannt. Da die derzeit vorgeschlagenen Einrichtungen zur biochemischen Analyse noch nicht alle Anforderungen der Praxis erfüllen, ist es insbesondere Aufgabe der Erfindung, das System so zu verbessern, bei dem eine weitestgehend automatisierte und reproduzierbare Auswertung möglich ist. Insbesondere soll dabei eine unmittelbare Anzeige des Ergebnisses auf einem Display bzw. eine Weitergabe der Daten in ein Computersystem gewährleistet sein. Dafür soll ein geeignetes Betriebsverfahren angegeben werden.

Die Aufgabe ist bei einem System der eingangs genannten Art durch Gesamtheit der Merkmale des Patentanspruches 1 gelöst. Weiterbildungen sind in den Unteransprüchen angegeben.

Das Betriebsverfahren zur Durchführung einer Auswertung einer Probe in einer einmal verwendbaren Cartridge mit Hilfe des Steuergerätes in einem System der eingangs genannten Art ist im Patentanspruch 22 angegeben.

Grundlage der Erfindung ist also ein gegenüber dem Stand der Technik verbessertes Konzept: Gemäß diesem Konzept realisierte Konstruktion eines Geräts ist erfindungsgemäß zur vollautomatischen Prozessierung und Auswertung von molekulardiagnostischen Analysen mit Einweg-Cartridges (Lab-on-a-Chip), insbesondere zur Steuerung und Auslesung von Analyseprozessen innerhalb von Cartridges in Form von modifizierten Scheckkarten, ausgebildet.

Bei der Offenbarung weist das Steuergerät insbesondere folgende Merkmale auf:
- eine Cartridge-Aufnahme zur Aufnahme, Verrieglung, Entriegelung und Freigabe der Cartridge,
- Mittel zur korrekten Positionierung der Cartridge im Lesegerät,
- Sensoren zur Erkennung der Cartridge, insbesondere einer richtigen Cartridge, z.B. Endschalter, Bar-Code,
- ein Wasser-Reservoir zum Verdünnen der Probe, Auflösen von Trockenreagenzien der Cartridge, Durchführung von Spülvorgängen, wobei das Wasser-Reservoir mittels Durchstechen eines Septums durch eine spitze Hohlnadel fluidisch kontaktierbar ist,
- ein Sterilfilter-Belüftungs-Ventil zum Belüften des Wasser-Reservoirs bei Entnahme von Wasser,
- ein Netzteil,
- eine Pumpe zum Fördern kleinster Flüssigkeitsmengen, beispielsweise im Mikroliter-Bereich,
- Mittel zum Verteilen von Wasser über Ventile, z.B. Wasserführende Magnetventile,
- einen Drucksensor in der Wasserzuführung zur Kontrolle von fluidischen Vorgängen,
- wenigstens eine Schnittstelle zur Zuführung von Wasser in die Cartridge,
- Sensoren zur Erkennung der Befüllung von Durchflusskanälen auf der Cartridge, z.B. kapazitiven Sensoren mit Elektroden oberhalb und unterhalb der Cartridge,
- Mittel zum Verschließen und Öffnen von Entlüftungsöffnungen der Cartridge, z.B. Luft-führende Magnetventile,
- Mittel zum Verschließen und Öffnen von Kammern, insbesondere der PCR-Kammer, auf der Cartridge, z.B. auf eine Folie drückende Stößel,
- eine Kombination aus einem Magnet-Bead-Sammler und einer Thermostatisierungeinrichtung zum Einfangen von Magnet-Beads und zur Durchführung von Thermozyklisierungen für die PCR,
- eine Sandwich-Anordnung von zwei vorgenannten Einrichtungen zur schnellen Thermozyklisierung,
- Mittel zur Sicherstellung optimalen Wärmübertragung zwischen der Thermostatisierungseinrichtung und Cartridge, z.B. mittels eines über Federn definierten Anpressdrucks,
- Mittel zur elektrischen Kontaktierung eines elektrischen Detektionsmoduls, z.B. Kontaktstifte,
- eine integrierte Thermostatisierung für das Detektionsmodul,
- eine Steuer-, Mess- und Regel-Elektronik für alle Funktionen,
- einen Mikrocontroller zur Steuerung und Datenerfassung und
- ein Display zur Darstellung der Analysenergebnisse.

Beim zugehörigen Verfahren zum Betrieb des Steuer- und Auslesegerätes zur Auswertung einer mit einer Probe gefüllten Cartridge werden speziell für eine DNA-Analyse folgende Verfahrensschritte durchgeführt:
- die Cartridge-Aufnahme wird geöffnet und die Pumpe entnimmt bei geeigneter Ventilstellung Wasser aus dem Reservoir,
- das Steuergerät zeigt Betriebsbereitschaft an,
- ein Benutzer schiebt die Cartridge mit einer Probe, z.B. Blut, ein,
- die Analyse wird durch eine "one button"-Aktion oder automatisch, z. B. mittels eines Cartridge-Endschalters gestartet,
- bei geeigneter Ventilstellung wird Wasser über einen Wasser-Port in die Cartridge gepumpt um überschüssiges Probenmaterial auszuwaschen,
- die Entlüftungs-Ventile werden in geeigneter Weise betätigt, um über Zulassung bzw. Vermeidung einer Entlüftung das Befüllen von Cartridge-Kanälen zu erlauben bzw. zu verhindern,
- durch den Zufluss von Wasser wird die Probe verdünnt und in den Zellaufschlusskanal gepumpt, wobei Trockenreagenzien gelöst werden,
- es erfolgt eine Verweilzeit zum Zellaufschluss, wobei freigesetzte DNA an Magnet-Beads bindet,
- zeitnah werden zwei ELISA-Reagenz-Kanäle befüllt, wobei trockene ELISA-Reagenzien (Enymlabel und Substrat) gelöst,
- der Inhalt des Zellaufschlusskanals wird über Weiterpumpen von Wasser durch die PCR-Kammer gespült, wobei die Magnet-Beads mit DNA durch ein Magnetfeld zurückgehalten werden, der Rest wird in Abfallkanal gespült,
- die Ventile der PCR-Kammer werden verschlossen,
- die Thermozyklisierung wird gestartet, wobei trockene PCR-Reagenzien gelöst werden,
- eine vorgegebene Anzahl von Thermozyklen wird unter definierten Temperaturen durchgeführt (PCR),
- die Ventile der PCR-Kammer werden nach Abschluss der PCR geöffnet,
- bei geeigneter Ventilstellung wird über einen weiteren Wasserport Wasser in die Cartridge gepumpt und das PCR-Produkt von der PCR-Kammer in eine Detektionskammer gespült,
- die Detektionskammer wird thermostatisiert, wobei eine Hybridisierung der DNA mit Fängermolekülen stattfindet,
- etwaig zwischen dem PCR-Produkt und den gelösten ELISA-Reagenzien vorhandene Luft wird durch geeignete Stellung von Wasser-Port- und Entlüftungs-Ventilen in einen Abfallkanal gepumpt,
- das gelöste Enzymlabel wird durch geeignete Stellung von Wasser-Port- und Entlüftungs-Ventilen über die Detektionskammer in eine weiteren Abfallkanal gepumpt,
- das gelöste Substrat wird durch geeignete Stellung von Wasser-Port- und Entlüftungs-Ventilen über die Detektionskammer in eine weiteren Abfallkanal gepumpt,
- die elektrische Detektion wird gestartet,
- es wird z.B. eine SNP-Analyse durch Aufnahme von Schmelzkurven durchgeführt.

Alternativ zu vorstehendem Verfahrensablauf ist auch eine Protein-Analyse möglich, wobei kein Zellaufschluss und keine PCR notwendig sind. Dabei werden die gesuchten Proteine aus der Probe mittels Magnet-Beads isoliert und in der Detektionskammer nachgewiesen. Analog sind Haptene nachweisbar.

Mit dem erfindungsgemäßen Verfahren ist nunmehr ein durchgängig integrierter Analyseprozess durchgeführt. Darunter wird ein vollständig automatisierter Verfahrensablauf ohne manuellen Eingriff verstanden. Nach Einführen der Cartridge mit der Probe in das Steuergerät läuft der Probenbehandlungs- und Analyseprozess bis zum eigentlichen Nachweis der gesuchten Substanzen automatisch ab. Dies hat insbesondere den Vorteil einer hohen Reproduzierbarkeit und einer geringen Fehlerquote.

Besonders vorteilhaft ist beim erfindungsgemäßen System, dass das Steuergerät kompakt ausführbar ist und sowohl interne fluidische Schnittstellen als auch elektrische Schnittstellen hat. Zusätzlich sind Schnittstellen zur Anbindung an externe Computersysteme vorhanden. Dies können beispielsweise serielle und/oder drahtlose Schnittstellen sein. In einer bevorzugten Ausführungsform umfasst das Steuergerät eine Anschlussmöglichkeit für einen handelsüblichen PDA. Damit ist auf einfache Weise eine Anzeige in das Steuergerät integrierbar. In einer besonders bevorzugten Ausführungsform ist eine Anzeige- und Bedieneinheit fest in das Steuergerät integriert.

Mit dem erfindungsgemäßen System wird gegenüber den bisher vorgeschlagenen Geräten ein beachtlicher Fortschritt erreicht. Es ist nunmehr beispielsweise möglich, die gemessenen Daten unmittelbar anzuzeigen bzw. zur Auswertung weiterzuleiten.

Weitere Einzelheiten und Vorteile der Erfindung werden anhand des im Folgenden beschriebenen Ausführungsbeispiels im Zusammenhang mit den beigefügten Zeichnungen erläutert. Es zeigen:
- Figur 1: ein schematisches Übersichtsbild eines Analysegeräts,
- Figur 2: ein schematisches Übersichtsbild einer Cartridge,
- Figur 3: ein schematisches Ablaufdiagramm eines Analysever fahrens,
- Figur 4: ein schematisches Übersichtsbild der funktionellen Einheiten des Steuergeräts,
- Figur 5: ein schematisches Übersichtsbild der fluidischen Komponenten des Steuergeräts,
- Figur 6 und Figur 7: schematische Darstellungen einer Messeinrichtung für einen Füllstand,
- Figur 8: eine schematische Darstellung einer fluidischen Schnittstelle zwischen der Cartridge und dem Steuergerät,
- Figur 9: eine schematische Darstellung einer Entlüftungsschnittstelle zwischen der Cartridge und dem Steuergerät,
- Figur 10: eine schematische Darstellung einer PCR-Kammer der Cartridge und
- Figur 11: eine schematische Darstellung einer Detektionskammer der Cartridge.

Im Folgenden wird als ein Ausführungsbeispiel der Erfindung ein Analysegerät beschrieben. Das Analysegerät ist in Steuergerät und eine Cartridge aufgeteilt. Während die Cartridge vorzugsweise als Einwegartikel vorliegt, auf dem der eigentliche Analyseprozess abläuft, ist das Steuergerät mehrfach verwendbar und dient zur Steuerung des Analyseprozesses. Die Cartridge enthält lediglich passive Komponenten, die gesamte Steuerung geht vom Steuergerät aus.

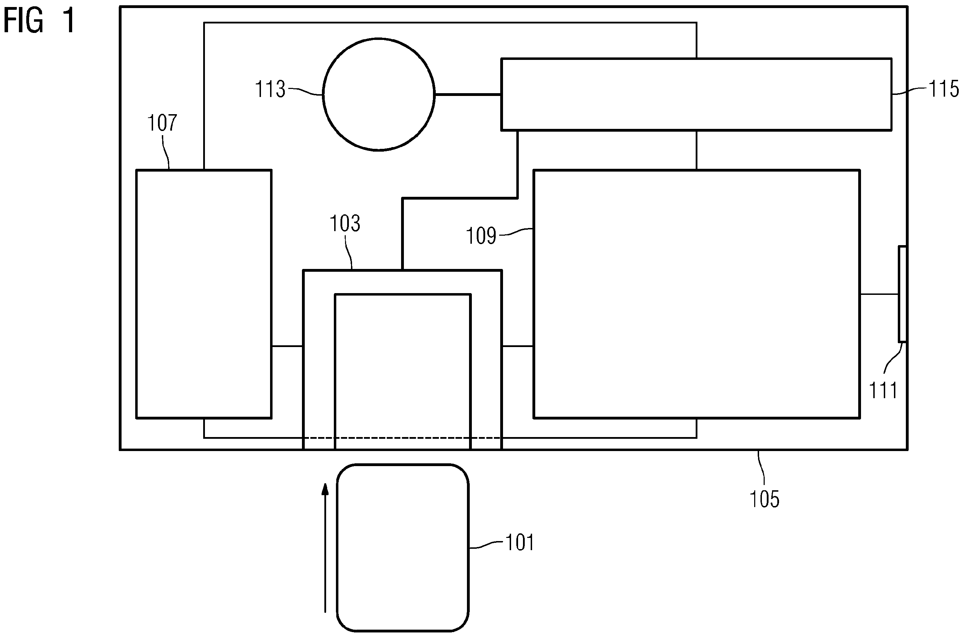

In Figur 1 ist der Aufbau des Analysegeräts gezeigt. Es besteht aus einer Cartridge 101, die in eine Cartridge-Aufnahme 103 eines Steuergeräts 105 eingeschoben werden kann. Die Cartridge-Aufnahme 103 ist mit einem Netzteil 107 verbunden, durch das sie mit Strom versorgt wird. Zur Übertragung von Daten des Analyseprozesses ist die Cartridge-Aufnahme 103 außerdem mit einer Elektronik 109 verbunden. Die Daten können von der Elektronik 109 über eine Schnittstelle 111 ausgegeben werden. Die Schnittstelle 111 ist beispielsweise als RS-232-Schnittstelle ausgebildet. Die Elektronik 109 ist zur Versorgung mit Strom mit dem Netzteil 107 verbunden.

Zur Durchführung des Analyseprozesses wird Wasser in der Cartridge benötigt. Dieses Wasser ist in einem Vorratsbehälter 113 gelagert. Der Vorratsbehälter 113 ist beispielsweise eine handelsübliche Flasche mit sterilem, destilliertem Wasser, die durch ein Septum verschlossen ist. Eine entsprechende Aufnahme für die Flasche im Steuergerät verfügt über eine Kanüle, die bei Einsetzen der Flasche das Septum durchsticht. Dadurch wird das Wasser zugänglich und ist über eine verbundene Pumpe 115 aus der Flasche entnehmbar. Die Pumpe 115 kann definierte Wassermenge aufnehmen und über eine Verbindung in die Cartridge-Aufnahme 103 pumpen. Über die Cartridge-Aufnahme 103 gelangt das Wasser in die Cartridge 101 und steht im Analyseprozess zur Verfügung. Die Pumpe 115 ist zur Stromversorgung mit dem Netzteil 107 verbunden.

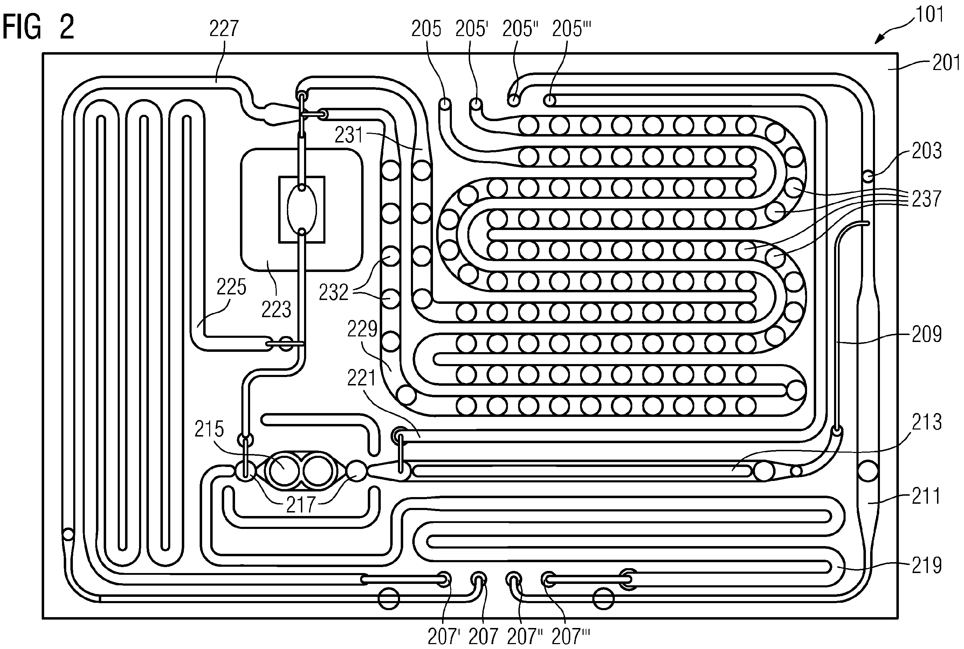

In Figur 2 ist die Cartridge 101 schematisch dargestellt, die im Einzelnen bereits in der nicht vorveröffentlichten WO 2006/042 838 A1 offenbart ist.

Die Cartridge 101 liegt in Form einer Kunststoffkarte 201 in der Größe einer Scheckkarte vor und umfasst eine Anzahl von Kanälen. Über einen Probenport 203 kann eine Probe in die Cartridge 101 einpipettiert werden. Über vier Wasserports 205, 205', 205" und 205''' kann Wasser in die Cartridge 101 eingebracht werden. Die Cartridge 101 verfügt außerdem über vier Entlüftungsports 207, 207', 207" und 207"', durch die Luft beim Füllen der Kanäle entweichen kann. Eine 1 und dient zum Abzweigen dieser?Dosierstrecke 209 umfasst ein Volumen von 1 Menge aus der einpipettierten Probe. Ein Probenabfallkanal 211 dient zur Aufnahme der übrigen Probe. Am Ende des Probenabfallkanals 211 liegt der Entlüftungsport 207"'. An die Dosierstrecke 209 schließt sich ein Zellaufschlusskanal 213 an, in dem Reagenzien für den Zellaufschluss in trockener Form gelagert sind. Erst durch das Einbringen der Probe in verdünnter Form werden die Reagenzien gelöst und ein Zellaufschluss findet statt. Durch den Zellaufschluss wird in den Zellen enthaltene DNA freigesetzt, die an ebenfalls im Zellaufschlusskanal 213 vorhandene Magnet-Beads binden.

Der Zellaufschlusskanal 213 ist mit einer PCR-Kammer 215 verbunden, in der eine PCR durchführbar ist. Die PCR-Kammer 215 ist mittels zweier Ventile 217 verschließbar. In der PCR-Kammer 215 befinden sich die benötigten Reagenzien ebenfalls in trockener Form. Hinter der PCR-Kammer 215 ist ein PCR-Abfallkanal 219 angeordnet, der mit dem Entlüftungsport 207" verbunden ist. An einem Ende des Zellaufschlusskanals 213 mündet ein Wasserkanal 221 ein, der mit dem Wasserport 205'' verbunden ist. Die PCR-Kammer 215 ist mit einer Detektionskammer 223 verbunden, in der die DNA mittels eines Sensorfelds nachweisbar ist. Die Detektionskammer 223 ist mit zwei Abfallkanälen 225 und 227 verbunden, die in den Belüftungsports 207' bzw. 207 enden. Zusätzlich ist die Detektionskammer 223 mit zwei ELISA-Reagenz-Kanälen 229 und 231 (ELISA: Enzym linked immuno sorbent assay) verbunden, die ihrerseits mit den Wasserports 205' bzw. 205 verbunden sind. In den ELISA-Reagenz-Kanälen 229 und 231 sind ELISA-Reagenzien 232 in trockener Form gelagert, die nach Lösen in Wasser in die Detektionskammer 223 gespült werden können.

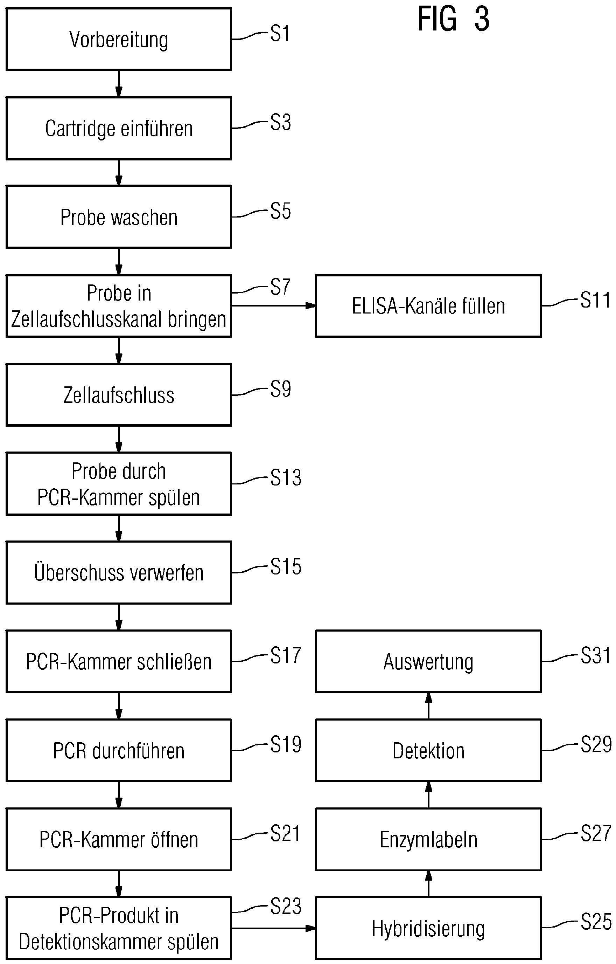

In Figur 3 ist ein schematisches Ablaufdiagramm eines Analyseprozesses gezeigt. In einem ersten Verfahrensschritt S1 wird die Cartridge vorbereitet. Dabei wird die Probe in die Cartridge einpipettiert und eventuell Wasser im Steuergerät bereitgestellt. Beim Einpipettieren sammelt sich die Probe im Probenabfallkanal 211 der Cartridge (bezogen auf Figur 2), wobei sich die Dosierstrecke 209 ebenfalls füllt. In einem zweiten Verfahrensschritt S3 wird die Cartridge in das Steuergerät eingeführt. Durch das Einführen der Cartridge werden im Auslesgerät befindliche Ventile mit den Wasserports 205 bis 205''' und den Entlüftungsports 207 bis 207''' verbunden, so dass Wasser in die Cartridge gebracht werden kann und Luft entweichen kann. Außerdem wird ein Schalter im Auslesgerät betätigt, worauf der Analyseprozess automatisch startet. Dabei wird über an sich bekannte Funktionselemente sichergestellt, dass die Cartridge in der richtigen Position ist und die richtige Orientierung hat. Außerdem wird erkannt, ob es sich um eine zum Steuergerät passende Cartridge handelt. Zu Beginn des Analyseprozesses innerhalb der Cartridge 101 wird der Wasserkanal 221 mit Wasser gefüllt, wobei der Wasserkanal 221 ebenfalls über den PCR-Abfallkanal 219 entlüftet. Dies vereinfacht den Verfahrensablauf, da im weiteren Ablauf die im Wasserkanal 221 vorliegende Luft nicht entweichen könnte.

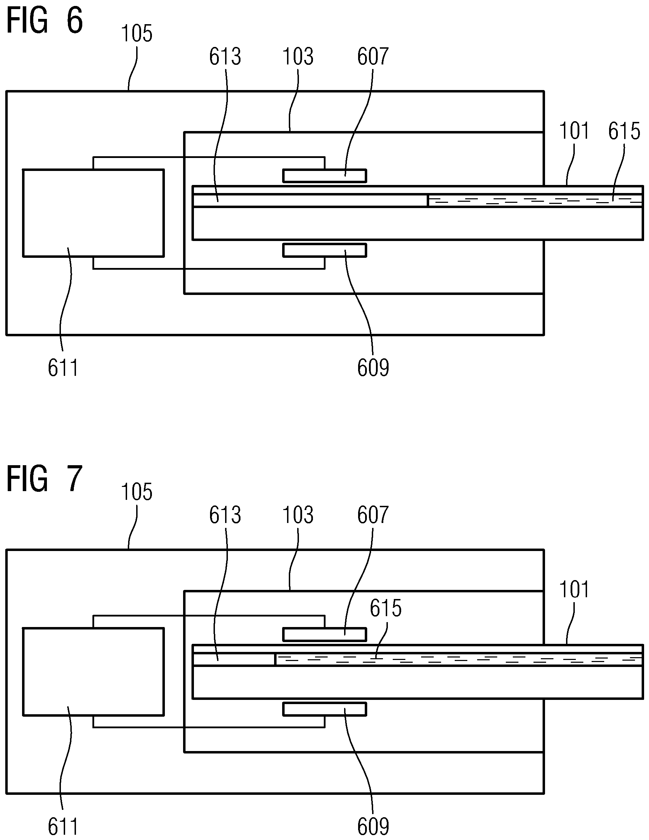

In einem dritten Verfahrensschritt S5 wird Wasser in den Wasserport 205''' gepumpt, so dass der in der Dosierstrecke 209 befindliche Teil der Probe isoliert wird. Dazu wird der ansonsten durch das Ventil verschlossene Entlüftungsport 207''' geöffnet. Sobald der Probenabfallkanal 211 gefüllt ist, wird der Entlüftungsport 207''' in einem vierten Verfahrensschritt S7 wieder verschlossen. Der Füllstand des Probenabfallkanals 211 wird mittels Füllstandsensoren gemessen, die im Steuergerät angeordnet sind. Die Funktionsweise wird anhand der Figuren 6 und 7 erläutert.

Durch das Verschließen des Entlüftungsports 207''' beginnt sich bei weiterem Einpumpen von Wasser Druck aufzubauen, so dass der in der Dosierstrecke 209 befindliche Teil der Probe in den Zellaufschlusskanal 213 gespült wird. Dazu wird das dem Entlüftungsport 207" zugeordnete Ventil im Steuergerät geöffnet. Es findet eine Verdünnung der Probe mit Wasser statt. Durch das Einbringen der verdünnten Probe lösen sich die trockenen Reagenzien im Zellaufschlusskanal 213 auf, woraufhin in einem fünften Verfahrensschritt S9 ein Zellaufschluss stattfindet. Dabei wird in den Zellen der Probe befindliche DNA freigesetzt. Die freigesetzte DNA bindet an im Zellaufschlusskanal 213 gelagerte Magnet-Beads an.

Gleichzeitig mit dem Zellaufschluss werden in einem sechsten Verfahrensschritt S11 die ELISA-Reagenz-Kanäle 229 und 231 mit Wasser gefüllt. Dazu werden die den Entlüftungsports 207' und 207 zugeordneten Ventile im Steuergerät geöffnet. Der Füllstand der ELISA-Reagenz-Kanäle 229 und 231 wird durch im Steuergerät angeordnete Füllstandssensoren gemessen.

Nachdem der Zellaufschluss abgeschlossen ist, wird in einem siebten Verfahrensschritt S13 die verdünnte Probe mit den Magnet-Bead-DNA-Komplexen durch die PCR-Kammer 215 gespült. Dazu wird wie schon zuvor Wasser durch den Wasserport 205''' gepumpt und der Entlüftungsport 207" geöffnet. Im Steuergerät ist oberhalb der PCR-Kammer 215 ein Magnet angeordnet, der die Magnet-Bead-DNA-Komplexe in der PCR-Kammer 215 zurückhält. Der Rest der verdünnten Probe wird in den PCR-Abfallkanal 219 gespült. In einem achten Verfahrensschritt S15 wird nochmals Wasser durch die PCR-Kammer 215 gepumpt, so dass die Magnet-Bead-DNA-Komplexe gereinigt werden. In einem neunten Verfahrensschritt S17 werden die Ventile 217 der PCR-Kammer 215 geschlossen. In einem zehnten Verfahrenschritt S19 wird die DNA der Magnet-Bead-DNA-Komplexe durch eine PCR vervielfältigt. Dazu befinden sich in der PCR-Kammer 215 entsprechende Reagenzien in trockener Form, die sich durch die Zugabe von Wasser lösen. Zur Manipulation der Temperatur in der PCR-Kammer 215 befinden sich im Steuergerät zwei Peltier-Einheiten. Nach Abschluss der PCR werden in einem elften Verfahrensschritt S21 die Ventile 217 der PCR-Kammer 215 geöffnet. In einem zwölften Verfahrensschritt S23 wird über den Wasserport 205'' Wasser in den Kanal 221 gepumpt, so dass das vorher im Kanal 221 befindliche Wasser in die PCR-Kammer 215 gespült wird. Der Kanal 221 enthält trocken gelagerte Salze, die so dem PCR-Produkt, also der vervielfältigten DNA zugesetzt werden. Gleichzeitig wird die vervielfältigte DNA von der PCR-Kammer 215 in die Detektionskammer 223 gespült. Die Entlüftungsports 207" und 207' sind dabei geschlossen, der Entlüftungsport 207 geöffnet.

In einem dreizehnten Verfahrensschritt 25 findet in der De tektionskammer 223 eine Hybridisierung zwischen dem PCR-Produkt und in der Detektionskammer 223 angeordneten Fängermolekülen statt. Zur Unterstützung der Hybridisierung wird die Temperatur in der Detektionskammer 215 über ein im Steuergerät angeordnetes Peltier-Element definiert eingestellt. In einem vierzehnten Verfahrensschritt S27 wird aus dem ELISA-Reagenz-Kanal 231 ein mit einem Kopplungs-Molekül verbundenes Label-Enzym (z.B. Streptavidin-Alkalische-Phosphatase, Streptavidin-Esterease oder Oligonukleotid-gekoppelte Esterase) durch die Detektionskammer gepumpt. Dazu wird Wasser durch den Wasserport 205 gepumpt und der Entlüftungsport 207' geöffnet. Durch das Label-Enzym wird die mit den Fängermolekülen verbundene DNA markiert. In einem fünfzehnten Verfahrensschritt S29 wird aus dem ELISA-Reagenz-Kanal 229 ein gelöstes Enzym-Substrat durch die Detektionskammer 215 gepumpt. Sowohl Enzym-Label, als auch Enzym-Substrat Kanal werden vor dem Fluten der Detektionskammer 231 über den Kanal 227 entlüftet, um ein Verschleppen von Luft in die Detektionskammer 223 zu vermeiden. Durch das Substrat kommt es in der Detektionskammer zu einem so genannten Redox-Cycling-Prozess, durch den die gebundene DNA nachgewiesen werden kann. Entsprechend findet in einem sechzehnten Verfahrensschritt S31 eine Auswertung der Messdaten statt.

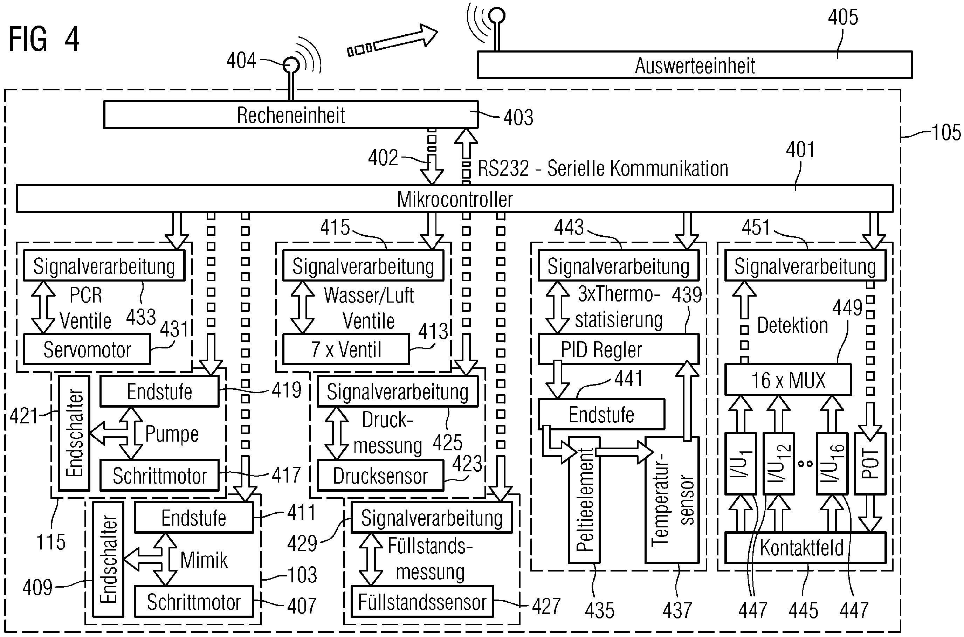

In Figur 4 sind die zentralen Komponenten des Steuergeräts 105 in einem Blockschaltbild dargestellt. Als zentrale Steuerung des gesamten Analyseprozesses dient ein Mikrocontroller 401, der über eine serielle RS232-Schnittstelle 402 mit einer Recheneinheit 403 verbunden ist. Diese Recheneinheit 403 ist beispielsweise ein handelsüblicher PDA und dient zur Aufnahme von Messdaten aus dem Analyseprozess. Die Recheneinheit 403 ist mit einem Funkmodul 404 ausgestattet, über das die Messdaten zu einer Auswerteeinheit 405 übertragbar sind. Das Funkmodul 404 kommuniziert mit dem Auswerteserver 405 beispielsweise per Infrarot, Bluetooth oder WLAN.

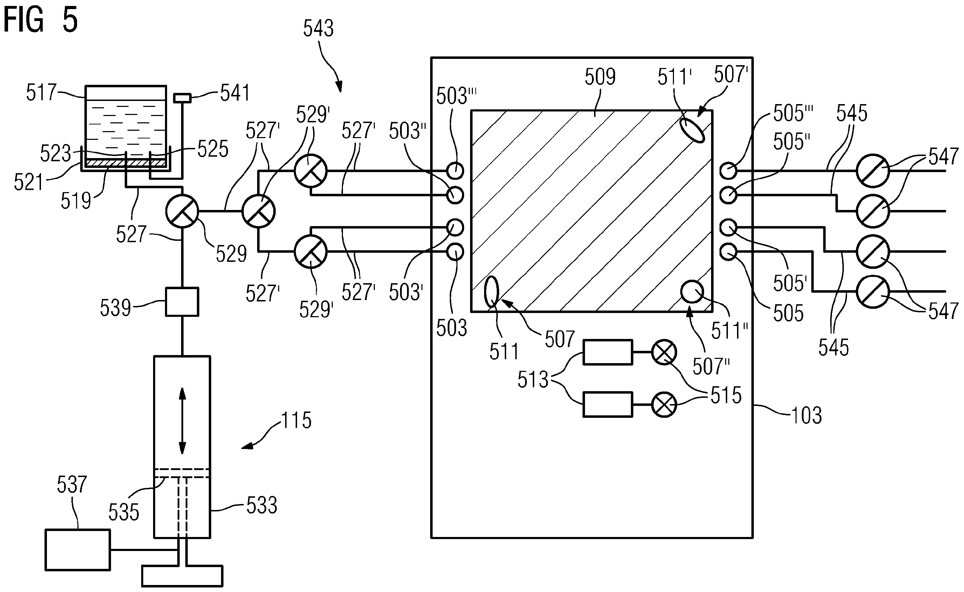

Der Mikrocontroller 401 steuert alle am Analyseprozess betei ligten Komponenten des Steuergeräts 105. Die Cartridge-Aufnahme 103 umfasst einen Schrittmotor 407, der bei Einschieben der Cartridge einen Teil der Cartridge-Aufnahme 103 bewegt, so dass die Cartridge korrekt aufgenommen wird. Dabei werden die Peltier-Elemente in Position gebracht. Ein Endschalter 409 unterbricht den Prozess bei vollständig eingeführter Cartridge. Durch den Einführprozess werden die Wasserports und die Entlüftungsports mit entsprechenden Kanälen im Steuergerät verbunden. In den Kanälen sind Ventile 413 angeordnet, die durch den Mikrocontroller 401 gesteuert werden. Eine Signalverarbeitungseinheit 415 dient zur Übermittlung der Steuersignale. Die Anordnung und Funktionsweise der Kanäle und Ventile wird anhand von Figur 5 erläutert.

Zur Bewegung des Wassers in den Kanälen wird die Pumpe 115 vom Mikrocontroller 401 gesteuert. Ihr Kolben wird durch einen Schrittmotor 417 bewegt, der wiederum mittels einer Endstufe 419 und einem Endschalter 421 gesteuert wird. Der Druck in der Pumpe 115 wird mittels eines Drucksensors 423 gemessen und über eine Signalverarbeitungseinheit 425 vom Mikrocontroller 401 ausgelesen.

Die Bewegung des Wassers in der Cartridge wird mittels Füllstandssensoren 427 überwacht, die über eine Signalverarbeitungseinheit 429 vom Mikrocontroller 401 ausgelesen werden.

Die Ventile der PCR-Kammer werden über einen Servomotor 431 und eine Signalverarbeitungseinheit 433 gesteuert. Zur Thermostatisierung der PCR-Kammer und der Detektionskammer dienen drei Peltier-Elemente 435 und Temperatursensoren 437, die über einen PID-Regler 439 und eine Endstufe 441 gesteuert und ausgelesen werden. Der PID-Regler 439 wird über eine Signalverarbeitungseinheit 443 vom Mikrocontroller 401 gesteuert.

Die durch das Redox-Cycling auf dem Sensorfeld erzeugten Signale werden über ein Kontaktierungsmodul 445 gemessen und über Ausleseschaltungen 447 und einen Multiplexer 449 an eine Signalverarbeitungseinheit 451 weitergegeben. Die Signalverarbeitungseinheit 451 ist mit dem Mikrocontroller verbunden. Dem Sensorfeld werden über das Kontaktierungsmodul 445 die notwendigen Steuerspannungen, wie z.B. eine Referenz- und eine Counter-Spannung eines Potenziostats zugeführt. Das Sensorfeld ist beispielsweise als ein CMOS-Sensor-Chip ausgeführt. Das Kontaktierungsmodul 445 hat in diesem Fall vorteilhafterweise die Anordnung wie bei einem handelsüblichen Smart-Card-Lesegerät.

In Figur 5 ist ein schematisches Übersichtsbild der fluidischen Komponenten des Steuergeräts dargestellt. Die Cartridge-Aufnahme 103 umfasst vier Wasserports 503, 503', 503'' und 503''', die bei eingeschobener Cartridge mit den deren Wasserports verbunden werden. Ebenfalls sind vier Entlüftungsports 505, 505', 505'' und 505''' vorgesehen, die mit den entsprechenden Entlüftungsports der Cartridge verbunden werden. Die Cartridge-Aufnahme 103 umfasst weiterhin drei Füllstandssensoren 507, 507' und 507'', deren Funktionsweise anhand der Figuren 6 und 7 erläutert wird. Sie arbeiten kapazitiv und bestehen aus zwei Kondensatorplatten. Zur Gewährleistung eines einfachen Aufbaus der Cartridge-Aufnahme 103 ist eine Kondensatorplatte 509 großflächig ausgebildet und wird für alle Füllstandssensoren 507, 507' und 507'' gemeinsam verwendet. Die anderen Kondensatorplatten 511, 511' und 511'' sind entsprechend getrennt ausgeführt. Die Füllstandssensoren 507 und 507' sind für jeweils zur Überwachung von zwei Kanälen der Cartridge ausgelegt und an entsprechenden Stellen der Cartridge-Aufnahme 103 angeordnet. Der Füllstandssensor 507 dient der Überwachung der beiden ELISA-Reagenz-Kanäle 229 und 231, während der Füllstandssensor 507' den Probenabfallkanal 211 und den PCR-Abfallkanal 219 überwacht. Der Füllstandssensor 507'' dient zur Überwachung des Abfallkanals 227.

In der Cartridge-Aufnahme 103 sind außerdem zwei Motoren 513 zur Steuerung zweier Aktuatoren 515 angeordnet. Die Aktuatoren 515 dienen zum Öffnen und Schließen der Ventile der PCR-Kammer auf der Cartridge.

Das für den Analyseprozess erforderliche Wasser stammt aus einer handelsüblichen Flasche 517, die mit einem Septum 519 verschlossen ist. Die Flasche 517 wird von einer Aufnahme 521 des Steuergeräts gehalten, in der zwei Kanülen 523 und 525 angeordnet sind. Bei Einführen der Flasche 517 in die Aufnahme 521 wird das Septum 519 mit den zwei Kanülen 523 und 525 durchstochen und das Wasser der Flasche 517 zugänglich gemacht. Die Kanüle 523 ist über eine Leitung 527 und ein Drei-Weg-Ventil 529 mit einer Pumpe 115 verbunden. Mittels der Pumpe 115 kann der Flasche 517 ein definiert einstellbares Volumen von Wasser entnommen werden. Die Pumpe 115 umfasst einen Hohlzylinder 533 mit einem beweglichen Kolben 535. Der Kolben 535 ist über einen Schrittmotor 537 definiert bewegbar. In der Leitung 527 ist ein Drucksensor 539 angeordnet, mittels dem der Druck in der Leitung 527 bestimmbar ist.

Die Kanüle 525 dient zur Belüftung der Flasche, da ansonsten bei der Entnahme von Wasser ein Unterdruck entstehen würde. Die Kanüle ist mit einem sterilen Filter 541 verbunden, über den Luft in die Flasche gelangen kann.

Das Drei-Weg-Ventil 529 ist über ein Leitungssystem 543 mit den vier Wasserports 503, 503', 503'' und 503''' verbindbar. Dazu sind mehrere Leitungen 527' und drei weitere Drei-Weg-Ventile 529' vorgesehen, durch die das Drei-Weg-Ventil 529 mit jedem der Wasserports 503, 503', 503'' und 503''' selektiv verbunden werden kann. Durch geeignetes Schalten der Drei-Weg-Ventile 529' kann so das Wasser aus dem Hohlzylinder 533 in einen der Wasserports 503, 503', 503" oder 503''' gepumpt werden. Der auftretende Druck im Leitungssystem 543 und der Leitung 527 wir dabei vom Drucksensor 539 erfasst.

Die vor Beginn des Analyseprozesses in den Kanälen der Cartridge vorhandene Luft muss beim Füllen mit Wasser aus der Cartridge gelangen können. Dazu sind die vier Belüftungsports 505, 505', 505'' und 505''' in der Cartridge-Aufnahme vorgesehen. Sie sind über Leitungen 545 mit Zwei-Weg-Ventilen 547 verbunden, die wahlweise geschlossen oder offen sein können. Beim Einbringen von Wasser in eine der Kanäle wird das entsprechende der Ventile 547 geöffnet, so dass Luft aus der Cartridge entweichen kann.

In den Figuren 6 und 7 ist die Funktionsweise der Füllstandssensoren gezeigt. In einer Schnittansicht ist in Figur 6 das Steuergerät 105 dargestellt. In die Cartridge-Aufnahme 103 ist eine Cartridge 101 eingeführt. In der Cartridge-Aufnahme 103 sind zwei Kondensatorplatten 607 und 609 oberhalb und unterhalb der Cartridge 101 angeordnet. Die Kondensatorplatten 607 und 609 sind mit einer Messeinheit 611 verbunden, mittels der die Kapazität des durch die Kondensatorplatten 607 und 609 gebildeten Kondensator bestimmbar ist.

In Figur 6 ist ein innerhalb der Cartridge 101 ausgeformter Kanal 613 teilweise mit Wasser 615 gefüllt. Bei der Darstellung in Figur 7 ist der Kanal 613 soweit gefüllt, dass Wasser 615' zwischen den Kondensatorplatten 607 und 609 steht. Dadurch ist die Kapazität des Kondensators im Vergleich zu dem in Figur 6 dargestellten Zustand verändert. Die Änderung wird mittels der Messeinheit 611 ermittelt und auf diese Weise der Flüssigkeitsstand im Kanal bestimmt.

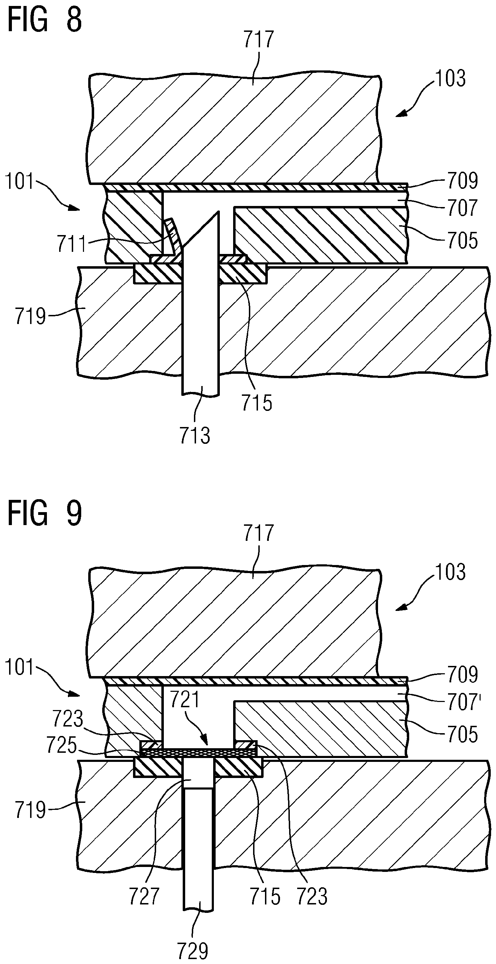

Figur 8 zeigt einen Schnitt durch einen Teil der Cartridge-Aufnahme 103 bei eingelegter Cartridge 101. Gezeigt ist die Kontaktierung zwischen den Wasserports des Steuergeräts 103 und der Cartridge 101. In einem Kunststoffkörper 705 der Cartridge 101 ist ein Kanal 707 gefräst. Der Kanal 707 ist mittels einer Klebefolie 709 abgedeckt, wobei die Klebefolie 709 auf den Kunststoffkörper 705 geklebt ist. Die Klebefolie 709 ist handelsüblich und mit allen in der Cartridge 101 ablaufenden Prozessen kompatibel. Am Wasserport der Cartridge 101 ist eine weitere Klebefolie 711 befestigt. Diese kann von einer in der Cartridge-Aufnahme 103 angeordneten Hohlnadel 713 durchstochen werden und so der fluidische Kontakt zwischen der Cartridge-Aufnahme 103 und der Cartridge 101 hergestellt werden. In der Cartridge-Aufnahme 103 ist eine Dichtung 715 angeordnet, die ein austreten von Flüssigkeiten verhindert. Die Dichtung 715 besteht vorzugsweise aus einem biochemisch kompatiblen Silikonmaterial um die Kontamination der Wasserports zu vermeiden.

Vor dem Einführen der Cartridge 101 in die Cartridge-Aufnahme 103 sind ein oberer Teil 717 und ein unterer Teil 719 der Cartridge-Aufnahme 103 weiter voneinander entfernt, als in der Figur 8 dargestellt. Dies ermöglicht ein komfortables Einführen der Cartridge 101. Sobald die Cartridge 101 vollständig eingeführt ist werden die beiden Teile 717 und 719 zusammengeführt und die Cartridge 101 in der Cartridge-Aufnahme 103 verriegelt. Gleichzeitig wird die Klebefolie 711 von der Hohlnadel 713 durchstochen.

Figur 9 zeigt einen Schnitt durch einen Teil der Cartridge-Aufnahme 103 bei eingelegter Cartridge 101 bei einem der Entlüftungsports. In der Cartridge 101 ist analog zu der Darstellung der Figur 8 in den Kunststoffkörper 705 ein Kanal 707' gefräst, der von der Klebefolie 709 abgedeckt ist. Um den Entlüftungsport 721 der Cartridge 101 ist eine Vertiefung in den Kunststoffkörper 705 gefräst, in der eine Klebeschicht 723 angeordnet ist. Auf der Klebeschicht 723 ist eine Teflon-Membran 725 befestigt. In dem unteren Teil 719 der Cartridge-Aufnahme 103 ist eine Dichtung 715 angeordnet. Außerdem ist ein Kanal 727 vorgesehen, der durch die Dichtung 715 verläuft und somit in Kontakt zur Teflon-Membran 725 steht. In dem Kanal 727 ist ein Schlauch 729 angeordnet, der mit den Entlüftungsventilen des Steuergeräts verbunden ist.

Durch die Teflon-Membran 725 wird sichergestellt, dass keine Flüssigkeit aus der Cartridge austreten kann. Dies dient der Vermeidung einer Kontamination des Steuergeräts, dass für eine Vielzahl von Analyseprozessen einsetzbar sein soll. Die in die Cartridge 101 eingebrachte Probe enthält im Allgemeinen infektiöses Biomaterial, durch das das Steuergerät bei Kontakt kontaminiert würde und somit für eine Durchführung weiterer Analyseprozess gereinigt oder ersetzt werden müsste. Die Teflon-Membran 725 ist durchlässig für Dampf und Gas, jedoch undurchlässig für Flüssigkeiten. Mittels des Drucksensors 533 des Steuergeräts wird sichergestellt, dass der Druck in der Cartridge 101 nicht zu groß wird und die Teflon-Mem-bran 725 oder die Klebefolie 709 beschädigt oder aufgerissen wird.

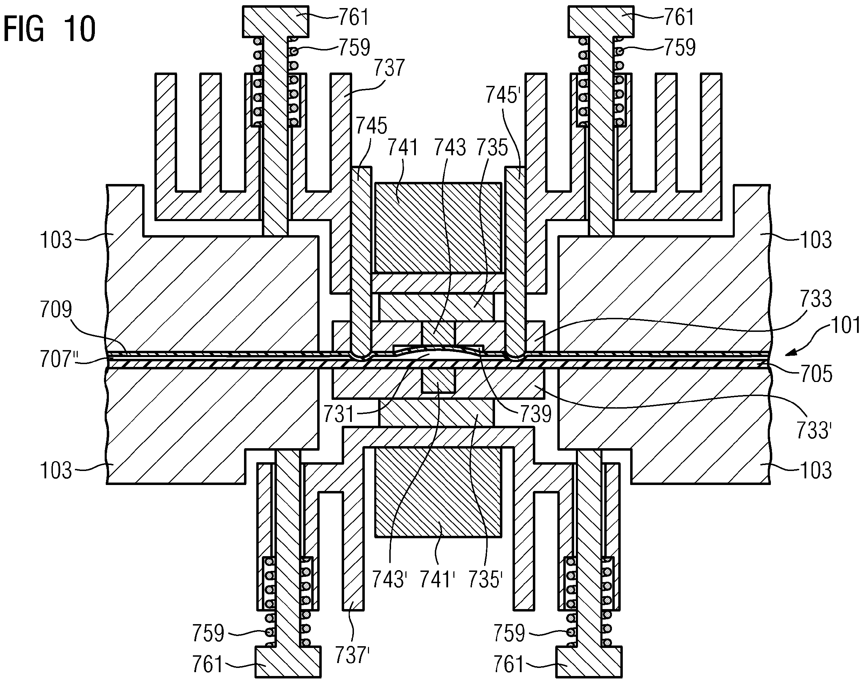

In Figur 10 ist ein Schnitt durch einen Teil der Cartridge-Aufnahme 103 bei eingelegter Cartridge 101 dargestellt. Analog zu den Figuren 8 und 9 ist ein Kanal 707'' in den Kunststoffkörper 705 gefräst. Der Kanal 707'' weist eine Verbreiterung auf, die als PCR-Kammer 731 dient. Der Kanal 707'' und die PCR-Kammer 731 sind durch die Klebefolie 709 abgedeckt.

Um einen schnellen Wärmetransport für die Thermozyklisierung bei der PCR zu gewährleisten, ist die PCR-Kammer 731 in einer geometrischen Dimension möglichst klein gehalten (ca. 1 mm bzw. Volumen < 20 µl) und die Wärmeübergänge werden in einer "Sandwich"-Thermostatisier-Anordnung so realisiert, dass lediglich geringe Flüssigkeitsschichten (einige 100 pm) ins thermische Gleichgewicht gebracht werden müssen. Die PCR-Kammer 731 befindet sich vorzugsweise in einem Gehäuse mit planparallelen Außenflächen (Kunststoffkörper 705 mit Aussparung und Klebefolie 709).

In der Cartridge-Aufnahme 103 sind oberhalb und unterhalb der PCR-Kammer 731 der Cartridge 101 zwei Wärmekopplungsplatten 733 und 733', beispielsweise aus Aluminium, angeordnet. Die beiden Wärmekopplungsplatten 733 und 733' sind gegen die Klebefolie 709 bzw. gegen den Kunststoffkörper 705 gedrückt und dienen zur Übertragung von Wärmeenergie zweier Peltier-Einheiten 735 und 735' in die PCR-Kammer 731 bei der Durchführung einer PCR. Die Peltier-Einheiten 735 und 735' stehen in thermischem Kontakt mit zwei Kühlkörpern 737 und 737', die zur Abführung von Wärme während der Abkühlvorgänge der PCR-Zyklen dienen. Sie verfügen über eine möglichst große Oberfläche, zur Ermöglichung eines effizienten Wärmeübergangs zur Umgebungsluft, gegebenenfalls unter Zuhilfenahme von Ventilatoren.

In der Wärmekopplungsplatte 733 ist eine Aussparung 739 ausgeformt. Durch die Aussparung 739 kann sich die Klebefolie 709 bei etwaigem Überdruck, beispielsweise während einer Temperaturerhöhung in einem PCR-Zyklus, auswölben.

Mittig oberhalb bzw. unterhalb der Kühlkörper 737 und 737' sind zwei Permanentmagneten 741 und 741' angeordnet. Das von den Permanentmagneten 737 und 737' erzeugte Magnetfeld dient der Zurückhaltung von Magnet-Beads, wenn diese durch den Kanal 707'' in die PCR-Kammer 731 gespült werden. So wird die an die Magnet-Beads gebundene DNA zur späteren Vervielfältigung durch die PCR in der PCR-Kammer 731 zurück gehalten.

Der von den beiden Permanentmagneten 741 und 741' erzeugte Feldgradient ist allerdings zu gering, um die Magnet-Beads effizient zurückzuhalten. Um dennoch einen hohen Feldgradienten in der PCR-Kammer 731 entlang der Durchflussrichtung der die Magnet-Beads enthaltenden Flüssigkeit zu erzielen, werden die wärmeleitfähigen aber magnetisch neutralen Wärmekopplungsplatten 733 und 733' mit kleinvolumigen Körpern 743 und 743' (ca. 5mm³) aus einem Material mit hoher Permeabilitätszahl µ(r), bei gleichzeitig noch guter Wärmeleitfähigkeit, vorzugsweise Permalloy (Ni-Fe) oder Mumetall (Ni/Fe/Cu/Mo), ausgestattet.

Die kleinvolumige Körper 743 und 743' bilden jeweils einen Magnetkern direkt an der Oberflächen der PCR-Kammer 731. Zwischen den Magnetkernen befindet sich nun direkt die PCR Kammer 731. Die Magnetkerne werden durch das Magnetfeld der Permanentmagneten 741 und 741' magnetisiert. Die Magnetfeldlinien werden über die Magnetkerne gebündelt, womit ein hoher Feldgradient erzeugt wird.

In dem Feldgradienten, der parallel zu den Außenwänden der PCR-Kammer 731 und Fließrichtung der Magnet-Beads enthaltenen Lösung angeordnet ist werden die Magnet-Beads hineingezogen, festgehalten und somit aufkonzentriert.

Die PCR-Kammer 731 ist mittels zweier Stößel 745 und 745' verschließbar. Die Stößel 745 und 745' sind mittels hier nicht dargestellter Federn und Nocken gegen die Klebefolie 709 drückbar, so dass die PCR-Kammer 731 verschlossen wird.

Die Kühlkörper 737 und 737' sind über Federn 759 mit Schrauben 761 verbunden. Die Schrauben 761 sind in die Cartridge-Aufnahme 103 geschraubt. Durch die Federn 759 werden die mit den Kühlkörpern 737 bzw. 737' verbundenen Wärmekopplungsplatten 733 bzw. 733' gegen die Klebefolie 709 bzw. den Kunststoffkörper 705 gedrückt, so dass ein guter thermischer Kontakt entsteht. Gleichzeitig wird ein thermischer Kontakt zwischen den Wärmekopplungsplatten 733 bzw. 733' und der Cartridge-Aufnahme 103 weitestgehend vermieden.

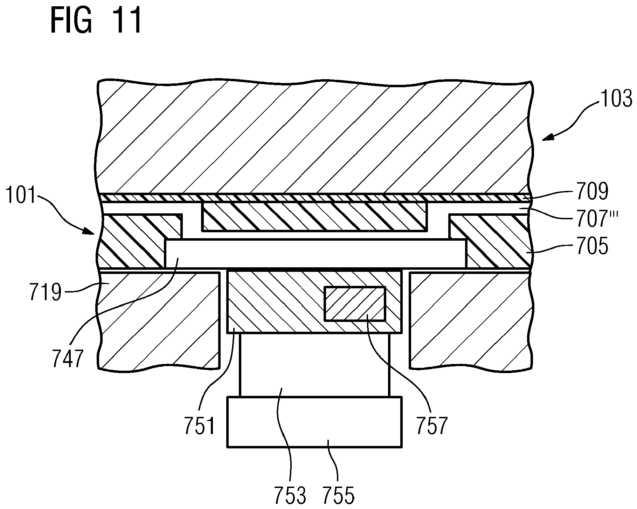

In Figur 11 ist ein Schnitt durch einen Teil der Cartridge-Aufnahme 103 bei eingelegter Cartridge 101 dargestellt. In den Kunststoffkörper 705 ist ein weiterer Kanal 707''' gefräst, der mit der Klebefolie 709 abgedeckt ist. In dem Kunststoffkörper 705 ist ein Chip-Modul 747 angeordnet, durch das die in der Probe enthaltene DNA nachweisbar ist. Auf dem Chip-Modul 747 kann beispielsweise ein Redox-Cycling-Prozess zum Nachweis der DNA stattfinden.

Zur Unterstützung der an der Oberfläche des Chip-Moduls 747 stattfindenden Hybridisierung zwischen der DNA und entsprechenden Fängermolekülen ist das Chip-Modul 747 über eine Wärmekopplungsplatte 751 mit einem Peltier-Element 753 thermisch gekoppelt. Das Peltier-Element 753 ist mit einer Kühleinheit 755 thermisch gekoppelt. Mittels eines in der Wärmekoppelplatte 751 angeordneten Temperatursensors 757 ist die Temperatur in der Umgebung des Chip-Moduls 747 kontrollierbar. So lassen sich beispielsweise SNP-Analysen über die Bestimmung von Schmelzkurven durchführen.

Außerhalb der Zeichnungsebene ist die aus der Wärmekopplungsplatte 751, dem Peltier-Element 753 und der Kühleinheit 755 gebildete Einheit mit dem unteren Teil 719 der Cartridge-Aufnahme verbunden. Dabei ist die Einheit federnd gelagert, so dass sie gegen die Cartridge 101 gedrückt wird und ein guter thermischer Kontakt zwischen der Wärmekopplungsplatte 751 und dem Chip-Modul 747 hergestellt wird.

Außerdem sind außerhalb der Zeichnungsebene gefederte Kontaktstifte innerhalb des unteren Teils 719 der Cartridge-Aufnahme 103 vorgesehen, die elektrische Signale des Chip-Moduls 747 zur Auswertung abgreifen.

Mit dem beschriebenen System aus Chipkarte zur Probenaufnahme und Auswertegerät mit Steuergerät zur Probenaufbereitung und -auswertung bei in das Auswertegerät eingebrachter Cartridge ist nunmehr eine praxisgerechte Bestimmung von DNA , Proteinen und auch Haptenen möglich.

## Patentansprüche

1. System zur integrierten und automatisierten DNA- oder Protein-Analyse, mit einer Cartridge (101) und einem Auswertegerät, wobei
- die Cartridge (101) zur Aufnahme einer Probe und verschiedener für die Analyse geeigneter Reagenzien in trockener und langzeitstabiler Form ausgebildet ist und
- das Auswertegerät ein Steuergerät (105) zur Ausführung eines Analyseprozesses bei in das Auswertegerät eingebrachter Cartridge (101) enthält,
und mit Mitteln zur Aufbereitung der Probe innerhalb der Cartridge (101), wozu aus einem Flüssigkeitsbehälter (113) Flüssigkeit zumindest den trockenen Reagenzien in der Cartridge (101) zugeführt werden kann zwecks Auflösung und anschließender Detektion nachzuweisender Bestandteile der Probe, wobei
- erste Mittel (103 - 113) zur Steuerung des Einbringens der Cartridge (101) in das Steuergerät (105) und des anschließenden in der Cartridge (101) ablaufenden Analyseprozesses einschließlich einer Bewegung und Thermostatisierung von in die Cartridge (101) eingebrachten Flüssigkeiten sowie
- zweite Mittel (401, 403, 405) zur Verarbeitung von durch den Analyseprozess gewonnenen Signalen vorhanden sind, und wobei
- die ersten und die zweiten Mittel (103 - 113, 401, 403, 405) im Steuergerät (105) derart aufeinander abgestimmt sind, dass der Analyseprozess der Probe vollintegriert durchführbar und die Signale unmittelbar ausgebbar sind,
wobei das Steuergerät (105) eine Cartridge-Aufnahme (103) zur Aufnahme, Verriegelung, Entriegelung und Freigabe der Cartridge (101) aufweist, wobei die Cartridge-Aufnahme (103) zwei Teile (717, 719) aufweist und wobei das System dazu ausgebildet ist, die beiden Teile (717, 719) zusammenzuführen und die Cartridge (101) in der Cartridge-Aufnahme (103) zu verriegeln, sobald die Cartridge (101) vollständig eingeführt ist.

2. System nach Anspruch 1, **dadurch gekennzeichnet, dass** zwischen Cartridge (101) und Steuergerät (105) Schnittstellen (101) für die Fluidik einerseits und eine Elektronik andererseits vorhanden sind.

3. System nach Anspruch 2, **dadurch gekennzeichnet, dass** das Steuergerät (105) wenigstens eine serielle und/oder eine drahtlose Schnittstelle (111) umfasst, über die die Signale und/oder wenigstens teilweise verarbeitete Signale übermittelbar sind.

4. System nach Anspruch 3, **dadurch gekennzeichnet, dass** die drahtlose Schnittstelle (111) als Bluetooth-System ausgebildet ist.

5. System nach Anspruch 1 oder 2, **dadurch gekennzeichnet, dass** ein Auswerteserver (405) vorhanden ist.

6. System nach einem der obigen Ansprüche, **dadurch gekennzeichnet, dass** ein PDA (403) vorhanden ist.

7. System nach einem der obigen Ansprüche, **dadurch gekennzeichnet, dass** Sensoren zur Erkennung einer korrekten Orientierung der Cartridge (101) in der Cartridge-Aufnahme (103) vorhanden sind.

8. System nach Anspruch 7, **dadurch gekennzeichnet, dass** Sensoren zur Erkennung eines korrekten Typs der Cartridge (101) in der Cartridge-Aufnahme (103) vorhanden sind.

9. System nach einem der obigen Ansprüche, **dadurch gekennzeichnet, dass** ein Netzteil (107) vorhanden ist.

10. System nach einem der obigen Ansprüche, **dadurch gekennzeichnet, dass** eine Pumpe (115) vorhanden ist.

11. System nach Anspruch 1, **dadurch gekennzeichnet, dass** Mittel (507, 507', 507") zur Erkennung der Befüllung von Kanälen (211, 219, 229, 231) auf der Cartridge (101) vorhanden sind.

12. System nach Anspruch 11, **dadurch gekennzeichnet, dass** die Mittel (507, 507', 507") zur Erkennung der Befüllung als kapazitive Sensoren mit Elektroden ausgeführt sind, wobei die Elektroden derart angeordnet sind, dass sie sich bei in das Steuergerät (105) eingeführter Cartridge (101) oberhalb und unterhalb der Cartridge (101) befinden.

13. System nach einem der obigen Ansprüche, **dadurch gekennzeichnet, dass** Mittel (217) zum Verschließen und Öffnen von Kammern (215) auf der Cartridge (101) vorhanden sind.

14. System nach Anspruch 13, **dadurch gekennzeichnet, dass** die Mittel (217) zum Verschließen und Öffnen der Kammern (215) aus bewegliche Stößel ausgeführt sind.

15. System nach einem der obigen Ansprüche, **dadurch gekennzeichnet, dass** eine kombinierte Einheit aus einem Magnet-Bead-Sammler zum Einfangen von Magnet-Beads und einer Thermostatisierungseinrichtung zur Durchführung von Thermozyklen für die Durchführung einer PCR im Steuergerät (105) vorhanden ist, wobei die Thermostatisierungseinrichtung zwei in einer Sandwich-Anordnung angeordnete Thermostatisierungsmodule umfasst, wobei sich die Cartridge (101) im eingeschobenen Zustand zwischen den Thermostatisierungsmodulen befindet.

16. System nach Anspruch 15, **dadurch gekennzeichnet, dass** Mittel (759) zur Erzeugung eines definierten Anpressdruckes zwischen den Thermostatisierungsmodulen und der Cartridge (101) vorhanden sind.

17. System nach einem der obigen Ansprüche, **dadurch gekennzeichnet, dass** Mittel zur elektrischen Kontaktierung eines elektrischen Chip-Moduls (747) vorhanden sind.

18. System nach Anspruch 17, **dadurch gekennzeichnet, dass** eine Thermostatisierungseinheit für das Chip-Modul (747) vorhanden ist.

19. System nach einem der obigen Ansprüche, **dadurch gekennzeichnet, dass** Steuer-, Mess- und Regel-Elektronik-Einrichtungen für die Funktionen vorhanden sind.

20. System nach einem der obigen Ansprüche, **dadurch gekennzeichnet, dass** ein Mikrocontroller (401) zur Steuerung und Datenerfassung vorhanden ist.

21. System nach einem der obigen Ansprüche, **dadurch gekennzeichnet, dass** eine Anzeige zur Darstellung der Analysenergebnisse vorhanden ist.

22. Verfahren zum Betrieb des Steuergerätes (105) in einem System gemäß einem der obigen Ansprüche, mit folgenden Verfahrensschritten:
- Einbringen einer Probe in eine Cartridge (101),
- Durchführen einer Analyse von in der Probe nachzuweisenden Bestandteilen in einem integrierten Prozess,
- Auswertung von aus dem Analysevorgang resultierenden Messsignalen.

## Claims

1. System for the integrated and automated analysis of DNA or protein, with a cartridge (101) and an evaluation device, wherein
- the cartridge (101) is designed for receiving a sample and several agents in dry and long-term stable form which are suitable for the analysis and
- the evaluation device contains a control device (105) for performing an analysis process with the cartridge (101) inserted in the evaluation device,
and with means for processing the sample within the cartridge (101), to which liquid from a liquid container (113) can be supplied to the dry reagents in the cartridge (101) for dissolving and subsequent detection of constituents to be detected of the sample, wherein
- first means (103 - 113) for controlling the insertion of the cartridge (101) into the control device (105) and the subsequent analysis process running in the cartridge (101) including a movement and thermostatic regulation of liquids introduced in the cartridge (101), as well as
- second means (401, 403, 405) for processing of signals obtained by the analysis process are present, and wherein
- the first and second means (103 - 113, 401, 403, 405) in the control device (105) are concerted in such a way that the analysis process of the sample can be performed in a fully integrated manner and the signals can be output directly,
wherein the control device (105) has a cartridge receptcacle (103) for receiving, locking, unlocking and releasing the cartridge (101), wherein the cartridge receptcacle (103) has two parts (717, 719) and wherein the system is designed to bring together the two parts (717, 719) and to lock the cartridge (101) in the cartridge receptacle (103) when the cartridge (101) is completely inserted.

2. System according to claim 1, **characterized in that**, between cartridge (101) and control device (105), on the one hand interfaces (101) for the fluidics and one the other hand electronics are present.

3. System according to claim 2, **characterized in that** the control device (105) comprises at least one serial and/or wireless interface (111) through which the signals and/or at least partly processed signals can be transmitted.

4. System according to claim 3, **characterized in that** the wireless interface (111) is designed as Bluetooth system.

5. System according to claim 1 or 2, **characterized in that** an evaluation server (405) is present.

6. System according to one of the above claims, **characterized in that** a PDA (403) is present.

7. System according to one of the above claims, **characterized in that** sensors for recognizing a correct orientation of the cartridge (101) in the cartridge receptacle (103) are present.

8. System according to claim 7, **characterized in that** sensors for recognizing a correct type of the cartridge (101) in the cartridge receptacle (103) are present.

9. System according to one of the above claims, **characterized in that** a power supply unit (107) is present.

10. System according to one of the above claims, **characterized in that** a pump (115) is present.

11. System according claim 1, **characterized in that** means (507, 507', 507") for recognizing the filling of channels (211, 219, 229, 231) on the cartridge (101) are present.

12. System according to claim 11, **characterized in that** the means (507, 507', 507") for recognizing the filling are designed as capacitive sensors with electrodes, wherein the electrodes are arranged in such a way that they are located above and below the cartridge (101) when the cartridge (101) is inserted in the control device (105).

13. System according to one of the above claims, **characterized in that** means (217) for closing and opening of chambers (215) on the cartridge (101) are present.

14. System according to claim 13, **characterized in that** the means (217) for closing and opening the chambers (215) are designed as movable plungers.

15. System according to one of the above claims, **characterized in that** a combined unit of a magnetic beads collector for catching magnetic beads and a thermostatic regulation device for performing thermocycles for performing a PCR in the control device (105) is present, wherein the thermostatic regulation device comprises two thermostatic regulation modules which are arranged in a sandwich arrangement, wherein the cartridge (101) is located between the thermostatic regulation modules in the inserted state.

16. System according to claim 15, **characterized in that** means (759) for producing a defined contact pressure between the thermostatic regulation modules and the cartridge (101) are present.

17. System according to one of the above claims, **characterized in that** means for the electric contacting of an electric chip module (747) are present.

18. System according to claim 17, **characterized in that** a thermostatic regulation unit for the chip module (747) is present.

19. System according to one of the above claims, **characterized in that** control, measuring and control electronics devices for the functions are present.

20. System according to one of the above claims, **characterized in that** a microcontroller (401) for control and data acquisition is present.

21. System according to one of the above claims, **characterized in that** a display for displaying the analysis results is present.

22. Method for the operation of a control device (105) in a system according to one of the above claims, with the following method steps:
- introducing a sample into a cartridge (101),
- performing an analysis of constituents to be detected in the sample in an integrated process,
- evaluation of measurement signals resulting from the analysis process.

## Revendications

1. Système d'analyse d'ADN ou de protéines intégrée et automatisée, comprenant une cartouche (101) et un appareil d'évaluation, dans lequel
- la cartouche (101) est conçue pour recevoir un échantillon et divers réactifs, sous une forme sèche et stable à long terme, appropriés pour l'analyse et
- l'appareil d'évaluation contient un appareil de commande (105) pour mettre en œuvre un processus d'analyse dans la cartouche (101) insérée dans l'appareil d'évaluation,
et comprenant des moyens de traitement de l'échantillon à l'intérieur de la cartouche (101) de telle sorte que, à partir d'un récipient de liquide (113),un liquide peut être acheminé au moins jusqu'aux réactifs secs dans la cartouche (101) dans le but de dissoudre et de détecter ultérieurement les composants de l'échantillon à détecter, dans lequel
- des premiers moyens (103-113), pour commander l'introduction de la cartouche (101) dans l'appareil de commande (105) et le processus d'analyse ultérieur se déroulant dans la cartouche (101), notamment un mouvement et une thermostatisation des liquides introduits dans la cartouche (101), et
- des seconds moyens (401, 403, 405), pour traiter des signaux obtenus par le processus d'analyse, sont présents, et dans lequel
- les premier et second moyens (103-113, 401, 403, 405) sont coordonnés les uns avec les autres dans l'appareil de commande (105) de telle sorte que le processus d'analyse de l'échantillon peut être réalisé de manière complètement intégrée et que les signaux peuvent être émis directement,
dans lequel l'appareil de commande (105) comporte un récipient de cartouche (103) pour recevoir, pour verrouiller, pour déverrouiller et pour libérer la cartouche (101), dans lequel le récipient de cartouche (103) comporte deux parties (717, 719) et le système est conçu pour réunir les deux parties (717, 719) et pour verrouiller la cartouche (101) dans le récipient de cartouche (103) dès que la cartouche (101) est complètement insérée.

2. Système selon la revendication 1, **caractérisé en ce que**, entre la cartouche (101) et l'appareil de commande (105), des interfaces (101), d'une part pour la fluidique et d'autre part pour l'électronique, sont présentes.

3. Système selon la revendication 2, **caractérisé en ce que** l'appareil de commande (105) comprend au moins une interface (111) série et/ou sans fil par l'intermédiaire de laquelle les signaux et/ou des signaux au moins partiellement traités peuvent être transmis.

4. Système selon la revendication 3, **caractérisé en ce que** l'interface (111) sans fil est conçue sous la forme d'un système Bluetooth.

5. Système selon la revendication 1 ou 2, **caractérisé en ce qu'**un serveur d'évaluation (405) est présent.

6. Système selon l'une quelconque des revendications précédentes, **caractérisé en ce qu'**un PDA (403) est présent.

7. Système selon l'une quelconque des revendications précédentes, **caractérisé en ce que** des capteurs pour reconnaître une orientation correcte de la cartouche (101) sont présents dans le récipient de cartouche (103).

8. Système selon la revendication 7, **caractérisé en ce que** des capteurs pour reconnaître un type correct de cartouche (101) sont présents dans le récipient de cartouche (103).

9. Système selon l'une quelconque des revendications précédentes, **caractérisé en ce qu'**un bloc d'alimentation (107) est présent.

10. Système selon l'une quelconque des revendications précédentes, **caractérisé en ce qu'**une pompe (115) est présente.

11. Système selon la revendication 1, **caractérisé en ce que** des moyens (507, 507', 507") pour détecter le remplissage de canaux (211, 219, 229, 231) sur la cartouche (101) sont présents.

12. Système selon la revendication 11, **caractérisé en ce que** les moyens (507, 507', 507") de détection du remplissage sont conçus sous la forme de capteurs capacitifs à électrodes, dans lequel les électrodes sont disposées de telle sorte qu'elles se trouvent au-dessus et en dessous de la cartouche (101) lorsque la cartouche (101) est insérée dans l'appareil de commande (105).

13. Système selon l'une quelconque des revendications précédentes, **caractérisé en ce que** des moyens (217) de fermeture et d'ouverture de chambres (215) de la cartouche (101) sont présents.

14. Système selon la revendication 13, **caractérisé en ce que** les moyens (217) de fermeture et d'ouverture des chambres (215) sont constitués de poussoirs mobiles.

15. Système selon l'une quelconque des revendications précédentes, **caractérisé en ce qu'**une unité combinée d'un collecteur de billes magnétiques pour capturer des billes magnétiques et d'un dispositif de thermostatisation pour effectuer des cycles thermiques afin d'effectuer une PCR est présente dans l'appareil de commande (105), le dispositif de thermostatisation comprenant deux modules de thermostatisation agencés en sandwich, la cartouche (101) étant située, à l'état inséré, entre les modules de thermostatisation.

16. Système selon la revendication 15, **caractérisé en ce que** des moyens (759) sont présents pour générer une pression de serrage définie entre les modules de thermostatisation et la cartouche (101).

17. Système selon l'une quelconque des revendications précédentes, **caractérisé en ce que** des moyens pour établir un contact électrique avec un module de puce (747) électrique sont présents.

18. Système selon la revendication 17, **caractérisé en ce qu'**une unité de thermostatisation pour le module de puce (747) est présente.

19. Système selon l'une quelconque des revendications précédentes, **caractérisé en ce que** des dispositifs électroniques de commande, de mesure et de régulation sont présents pour les fonctions.

20. Système selon l'une quelconque des revendications précédentes, **caractérisé en ce qu'**un microcontrôleur (401) est présent pour la commande et l'acquisition de données.

21. Système selon l'une quelconque des revendications ci-dessus, **caractérisé en ce qu'**un affichage est présent pour afficher les résultats d'analyse.

22. Procédé de fonctionnement de l'appareil de commande (105) dans un système selon l'une quelconque des revendications précédentes, comprenant les étapes suivantes :
- l'introduction d'un échantillon dans une cartouche (101),
- mise en œuvre d'une analyse de composants à détecter dans l'échantillon dans un processus intégré,
- l'évaluation des signaux de mesure résultant du processus d'analyse.
